# FASCICULE DE BREVET EUROPEEN

(11) **EP 2 020 938 B1**
(45) Date de publication et mention de la délivrance du brevet: **18.05.2016**
(21) Numéro de dépôt: 07765967.0
(22) Date de dépôt: 30.05.2007
(51) Int. Cl.: A61B 17/70

(54) **DISPOSITIF DE FIXATION OSSEUSE**
VORRICHTUNG ZUR KNOCHENFIXATION
BONE FIXING DEVICE

(30) Priorité: 30.05.2006 FR 0604803
(43) Date de publication de la demande: 11.02.2009
(73) Titulaire: Arthroplastie Diffusion, 01800 Saint-Maurice-de-Gourdans (FR)
(72) Inventeur: MOULIN, Jean-Pierre, 69006 Lyon (FR); ROY, Christophe, 69001 Lyon (FR); FAURE, Alexis, 44300 Nantes (FR); RAKOVER, Jean-Patrick, 72700 Rouillon (FR); LE NAELOU, Simon, 44340 Bouguenais (FR); VERNET, Philippe, 72000 Le Mans (FR); CISTAC, Christian, 85000 La Roche Sur Yon (FR); BACON, Philippe, 35510 Cesson Sevigne (FR); BLAMOUTIER, Arnaud, 35510 Cesson-Sevigne (FR); ALLIOUX, Jean-Jacques, 44220 Coueron (FR); ATANASIU, Jean-Pierre, 86000 Poitiers (FR); PARENT, Henry-François, 49100 Angers (FR); GAGNA, Gilles, 72000 Le Mans (FR)
(74) Mandataire: Brédeville, Odile Marie
(86) Numéro de dépôt international: PCT/FR2007/000893
(87) Numéro de publication internationale: WO 2007/138190

(56) Documents cités:
- EP-A1- 1 210 914
- WO-A-99/65415
- WO-A2-96/32070
- DE-A1-102004 010 844
- US-A- 5 507 746
- US-A- 5 676 665
- US-A- 5 989 251
- US-A1- 2005 277 927

## Description

La présente invention porte sur un dispositif de fixation osseuse pour réduire, stabiliser ou fixer des os, ainsi que sur un ensemble comprenant un tel dispositif et une tige de liaison destinée à relier entre eux plusieurs os ou fragments d'os.

Les dispositifs de fixation des os peuvent être internes, sous la forme d'implants mis en place en totalité dans le corps du patient, ou encore externes, avec toutefois des moyens d'ancrage fixés dans les os à traiter.

Les fixateurs externes à ancrage osseux comprennent des fiches ou broches, vissées ou traversant l'os ou le fragment d'os à fixer. Des éléments de liaison, comme par exemple une tige de liaison, positionnés à l'extérieur du corps font appel à des systèmes de blocage à serrage vissé comme des écrous ou des vis. De tels dispositifs sont fastidieux à mettre en oeuvre par leur complexité. De plus leur réglage est délicat. Ainsi, lors du vissage de la tige de liaison, des à-coups peuvent survenir et créer un jeu au site d'ancrage dans l'os, fragilisant la fixation.

Les fixateurs internes ou encore implants peuvent prendre la forme de clous ou broches positionnées longitudinalement, à l'intérieur de l'os ou des fragments d'os à fixer. Toutefois, de tels dispositifs ne permettent de traiter que les os longs.

Les implants peuvent également se présenter sous la forme de plaques percées d'orifices recevant des vis de fixation à l'os. Toutefois, de tels dispositifs n'offrent pas une bonne facilité d'alignement et/ou de positionnement des os ou fragments d'os : en effet, leur position est dictée par celle des orifices.

Les implants peuvent alternativement se présenter sous la forme de points d'ancrage reliés par des barres, tiges ou plaques positionnées à l'intérieur du patient. Ce type de fixateur interne présente toutefois une mise en oeuvre et un assemblage in situ complexes et peu rapides.

Il existe donc le besoin de fixateurs externes ou internes, faciles à mettre en oeuvre, de façon rapide, tout en étant fiables et en assurant une fixation optimale.

La présente invention vise à remédier à ce besoin en proposant un dispositif de fixation, pouvant être utilisé comme fixateur externe ou interne, particulièrement facile à mettre en place et assurant une excellente fixation.

La présente invention porte sur un dispositif de fixation osseuse apte à immobiliser une tige de liaison destinée à relier au moins un fragment d'os à un autre, ledit dispositif comprenant :
- au moins un moyen d'ancrage destiné à être fixé sur ou dans ledit fragment d'os,
- au moins une pièce réceptacle, solidaire dudit moyen d'ancrage, destinée à recevoir au moins une partie de ladite tige de liaison,
- des moyens de blocage agencés pour bloquer ladite partie de ladite tige de liaison au sein de ladite pièce réceptacle,
lesdits moyens de blocage comprenant au moins un élément solide présentant un axe longitudinal, ledit élément solide comprenant au moins une surface inclinée apte, sous l'action d'une poussée dirigée selon l'axe longitudinal dudit élément solide, à créer par friction, un coincement progressif de ladite partie de tige de liaison dans ladite pièce réceptacle, jusqu'au blocage complet de ladite partie de tige de liaison dans ladite pièce réceptacle.

Le dispositif selon l'invention permet un blocage particulièrement aisé et fiable de la tige de liaison. En particulier, le dispositif selon l'invention permet de s'affranchir de l'étape de vissage nécessaire avec les dispositifs de l'art antérieur.

Grâce à la surface inclinée de son moyen de blocage, le dispositif selon l'invention peut être mis en oeuvre par une simple poussée selon l'axe longitudinal de son moyen de blocage. Le coincement de la tige de liaison se fait de façon progressive, doucement, sans à-coups. On évite ainsi de provoquer une déstabilisation du moyen d'ancrage au niveau de l'os ou du fragment d'os considéré. Aucun jeu ne se crée au niveau de l'ancrage du dispositif dans ou sur l'os ou fragments d'os. La fixation est ainsi assurée de manière plus fiable qu'avec les dispositifs de l'art antérieur.

Selon l'invention,
- ladite pièce réceptacle est en forme de U, ladite partie de tige de liaison étant reçue au fond du U, la paroi de chaque branche du U étant pourvue d'un orifice,
- ledit élément solide est sous la forme d'une clavette dont la section transversale croît d'une première extrémité, appelée extrémité fine, à une deuxième extrémité, appelée extrémité large, le diamètre du cercle dans lequel est inscrit la section transversale de ladite extrémité large étant strictement supérieur au diamètre du cercle inscrit dans ledit orifice,
- ladite clavette étant apte à être introduite par son extrémité fine, successivement dans les orifices des parois des branches du U de ladite pièce réceptacle, perpendiculairement à ladite tige de liaison, et à être poussée selon son axe longitudinal jusqu'à entrer en contact avec ladite tige de liaison et créer une friction entre ladite tige de liaison et le fond de la pièce réceptacle,
de telle sorte que, en position de blocage, ladite clavette traverse chaque orifice de chaque branche du U de ladite pièce réceptacle et maintient ladite partie de tige de liaison bloquée contre le fond de ladite pièce réceptacle.

De préférence, le périmètre de la section transversale de ladite clavette présente une forme choisie parmi un cercle, un polygone, ou une combinaison d'arcs de cercle et/ou de segments.

Dans une forme de réalisation de l'invention, ladite clavette présente, sur sa partie destinée à être en contact avec ladite tige de liaison en position de blocage, des structures en creux ou en reliefs, telles que des aspérités, des rainures et/ou des hélices, aptes à sécuriser le blocage de ladite tige de liaison au sein de ladite pièce réceptacle.

Selon l'invention, les moyens de blocage sont solidaires dudit dispositif. Ainsi, le dispositif selon l'invention peut être fourni prêt à l'emploi avec son moyen de blocage déjà installé. Un tel dispositif permet un gain de temps pour le chirurgien qui n'a qu'à pousser sur le moyen de blocage, par exemple la clavette, déjà présent sur le dispositif, pour fixer la tige de liaison. L'opération de fixation est ainsi grandement facilitée et peut être exécutée extrêmement rapidement.

Selon l'invention, ladite clavette présente, en deçà de son extrémité fine, une gorge longitudinale s'étendant jusqu'à son extrémité large, apte à coopérer avec un ergot ménagé dans l'orifice de la paroi d'une première branche du U, afin de permettre le déplacement de ladite clavette vers l'orifice de la paroi de la deuxième branche du U et d'empêcher le retour complet de ladite clavette vers l'orifice de la paroi de la première branche du U et ainsi sa désolidarisation dudit dispositif.

Dans une forme de réalisation de l'invention, dans une configuration dite mobile, ladite pièce réceptacle est articulée sur ledit moyen d'ancrage, de manière à permettre une orientation angulaire contrôlable dans les trois directions entre ledit moyen d'ancrage et ladite tige de liaison.

Dans une forme de réalisation de l'invention, ladite pièce réceptacle présente un fond conique percé d'un trou axial,
- ledit moyen d'ancrage est sous la forme d'une vis comprenant une tête sphérique de diamètre strictement supérieur au diamètre dudit trou axial, ladite tête sphérique étant engagée dans le fond conique.

Avantageusement, ledit fond conique forme un cône d'angle α compris entre 15° et 60°. Un tel angle permet d'augmenter le serrage de la vis lors de la mise en pression. Il permet également la réversibilité du blocage. Le chirurgien peut facilement desserrer le moyen de blocage et orienter différemment la vis et donc la tige de liaison.

Dans une forme de réalisation de l'invention, lesdites branches du U sont aptes à se défléchir entre une position non fléchie au repos et une position défléchie sous contrainte, afin d'autoriser, sous contrainte, le passage de la tige de liaison vers le fond de la pièce réceptacle, et d'empêcher, au repos, la sortie de ladite tige de liaison du fond de la pièce réceptacle.

Dans une forme de réalisation de l'invention, ledit moyen d'ancrage est sous la forme d'une vis allongée, et l'axe longitudinal du U de ladite pièce de réceptacle est sensiblement parallèle à, et de préférence se confond avec, l'axe longitudinal A de ladite vis allongée.

Dans une autre forme de réalisation de l'invention, ledit moyen d'ancrage est sous la forme d'une vis allongée, et l'axe longitudinal du U de ladite pièce de réceptacle est sensiblement perpendiculaire avec l'axe longitudinal A de ladite vis allongée.

La présente invention porte également sur un ensemble pour fixation osseuse caractérisé en ce qu'il comprend au moins un dispositif tel que décrit ci-dessus et une tige de liaison.

Dans une forme de réalisation de l'invention, ladite tige de liaison présente une section transversale sensiblement en forme de croissant, ladite tige étant galbée. De préférence, une telle tige est galbée en per-opératoire afin d'adapter sa forme à la morphologie du patient.

Dans une autre forme de réalisation de l'invention, ladite tige de liaison est rectiligne et présente une section transversale en forme de polygone.

Les avantages de la présente invention vont maintenant ressortir de la description ci-après accompagnée des dessins annexés dans lesquels :
- la figure 1 est une vue en perspective d'un exemple d'un dispositif de fixation osseuse, position bloquée,
- la figure 2 est une vue en perspective d'un exemple d'un moyen de blocage d'un dispositif,
- la figure 3 est une vue en perspective d'une variante d'un moyen de blocage d'un dispositif,
- la figure 4 est une vue en perspective d'une autre variante d'un moyen de blocage d'un dispositif,
- la figure 5 est une vue en coupe du moyen de blocage du dispositif selon les figures 6 à 8,
- la figure 6 est une vue en perspective éclatée d'une forme de réalisation d'un dispositif selon l'invention,
- la figure 7 est une vue en perspective du dispositif selon figure 6, en position ouverte,
- la figure 8 est une vue en perspective du dispositif selon figure 6, en position bloquée,
- la figure 9 est une vue en perspective d'un exemple d'un dispositif,
- la figure 10 est une vue en coupe de la pièce réceptacle du dispositif selon la figure 9,
- la figure 11 est une vue en perspective d'un exemple d'un dispositif,
- la figure 12 est une vue en perspective de la fixation de trois fragments d'os à l'aide de trois dispositifs selon l'invention,
- la figure 13 est une vue en perspective d'une tige de liaison d'un ensemble selon l'invention,
- la figure 14 est une variante de la tige de liaison de la figure 13.

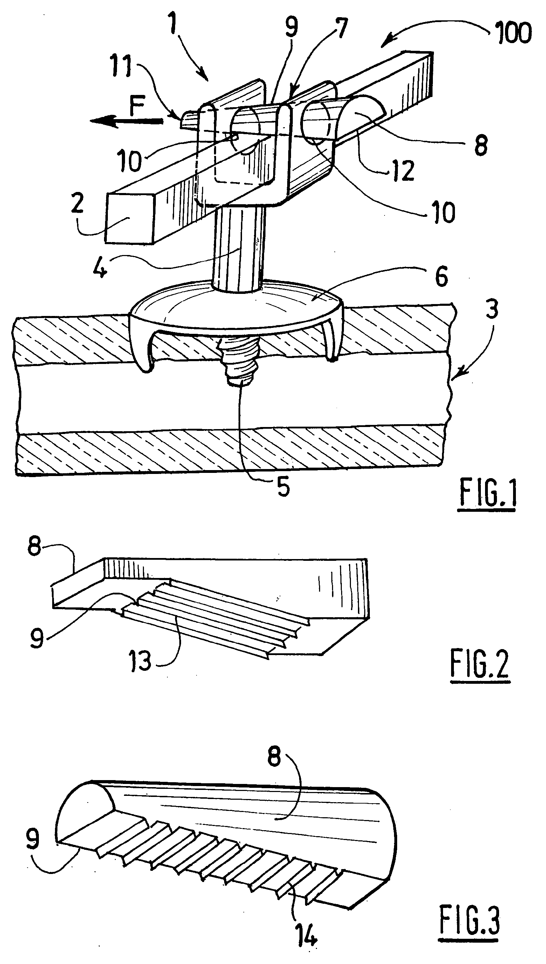

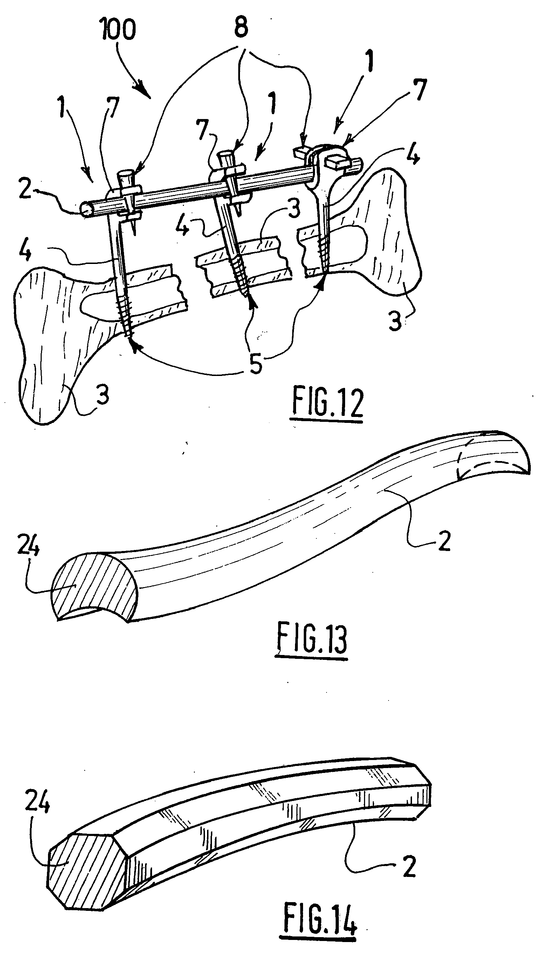

Sur la figure 1 est représenté un ensemble 100 comprenant un dispositif 1, pour immobiliser une tige 2 de liaison destinée à relier plusieurs os ou fragments d'os 3 entre eux. Par exemple, cette tige 2 de liaison peut relier deux vertèbres entre elles ou encore plusieurs segments d'os, par exemple d'un même os (voir figure 12).

Le dispositif 1 de la figure 1 comporte un moyen d'ancrage sous la forme d'une vis 4 d'ancrage dont l'extrémité filetée 5 est fixée, par exemple par vissage, dans l'os 3. Sur l'exemple de la figure 1, un moyen de fixation sous la forme d'une plateforme 6 munie de griffes s'ancrant dans l'os 3 est également présent pour renforcer l'ancrage du dispositif 1 dans l'os 3.

Le dispositif de la figure 1 comporte également une pièce réceptacle 7, solidaire du moyen d'ancrage 4, cette pièce réceptacle recevant au moins une partie de la tige 2 de liaison. Sur l'exemple représenté, la pièce réceptacle 7 est en forme de U, la paroi de chaque branche du U étant pourvue d'un orifice 10. De préférence, les deux orifices 10 sont identiques. La tige de liaison 2 est reçue au fond du U. Sur l'exemple représenté, l'axe longitudinal du U se confond avec l'axe longitudinal de la vis 4 d'ancrage.

Le dispositif de la figure 1 est en position bloquée : ainsi, la tige de liaison 2 est bloquée au sein de la pièce réceptacle 7 par des moyens de blocage. Dans l'exemple représenté, ces moyens de blocage comprennent un élément solide sous la forme d'une clavette 8, de forme sensiblement allongée et présentant une surface inclinée 9, dirigée substantiellement selon l'axe longitudinal de la clavette 8. Du fait de sa surface inclinée 9, la clavette 8 présente une section transversale variable, qui croît d'une de ses extrémités à l'autre. L'extrémité de plus petite section transversale 11 sera appelée extrémité fine, et l'extrémité 12 de plus grande section transversale sera appelée large. Le diamètre du cercle dans lequel est inscrit la section transversale de l'extrémité large 12 de la clavette 8 est strictement supérieur au diamètre du cercle inscrit dans l'orifice 10.

Sur l'exemple représenté figure 1, le périmètre de la section transversale de la clavette 8 présente la forme d'une combinaison d'un demi-cercle et d'un segment, la clavette 8 ayant sensiblement la forme d'une demi portion de cône.

Comme il ressort de la figure 1, la mise en place de la clavette 8 et le blocage de la tige 2 de liaison se fait de la façon suivante : le dispositif 1 est fourni au chirurgien dans une position ouverte, par exemple sans la clavette 8. Le chirurgien place la tige de liaison 2 au fond du U de la pièce réceptacle 7 puis il introduit la clavette 8 dans un premier orifice 10 par l'extrémité fine 11 de la clavette 8, perpendiculairement à la tige 2 de liaison, selon la flèche F représentée sur la figure 1. Le chirurgien pousse ensuite sur l'extrémité large 12 de la clavette 8, selon l'axe longitudinal de la clavette, et l'extrémité fine 11 de la clavette traverse le deuxième orifice 10. Au cours de son déplacement selon la flèche F, la clavette 8 entre en contact avec la tige 2 de liaison et crée une friction entre la tige 2 de liaison et le fond de la pièce réceptacle 7. Au fur et à mesure de son avancement selon le sens de la flèche F, la surface inclinée 9 de la clavette 8 crée un coincement progressif de la tige 2 de liaison dans la pièce réceptacle 7. En fin d'opération, le chirurgien, éventuellement à l'aide d'un moyen de serrage, serre fermement la clavette 8 et la tige 2 de liaison est totalement bloquée au sein de la pièce réceptacle.

Ainsi, du fait de l'absence de vissage du moyen de blocage, l'opération de mise en place de la clavette 8 et de blocage de la tige 2 de liaison se fait en douceur, progressivement et sans à-coups. Il n'y a ainsi pas de risque de provoquer un à-coup au niveau du moyen d'ancrage, ici de la vis 4, et de créer un jeu dans l'os, qui déstabiliserait ensuite la fixation du dispositif et donc de la tige 2 de liaison.

Sur la figure 2, est représentée une clavette 8 dont le périmètre de la section transversale est un polygone sous la forme d'un rectangle. La clavette 8 de la figure 2 présente en outre sur sa surface inclinée 9, destinée à être en contact avec la tige 2 de liaison, des rainures longitudinales 13. Ces rainures longitudinales sont aptes à sécuriser le blocage de la tige 2 de liaison au sein de la pièce réceptacle 7.

Sur la figure 3 est représentée une variante de la clavette 8 de la figure 2, dont la surface inclinée 9, destinée à être en contact avec la tige 2 de liaison, comporte des rainures transversales 14. Ces rainures 14 augmentent la force du blocage de la tige 2 de liaison dans la pièce réceptacle 7

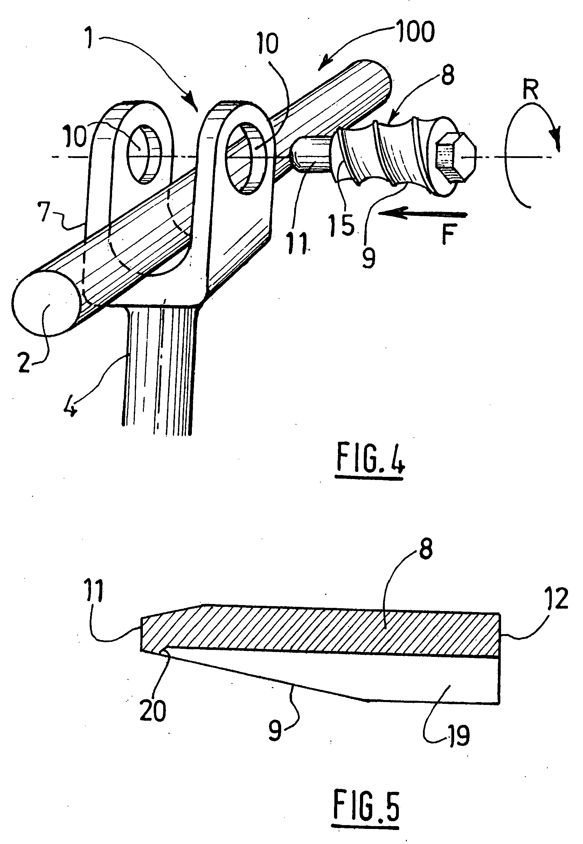

Sur la figure 4 est représenté partiellement un exemple d'un dispositif 1, muni d'une clavette 8 dont la surface inclinée 9 présente une hélice 15. Selon l'exemple de cette figure, le chirurgien, après avoir placé la tige 2 de liaison au fond de la pièce réceptacle 7, introduit la clavette 8 successivement au travers des deux orifices 10 par l'extrémité fine 11 de ladite clavette 8 en poussant cette dernière selon son axe longitudinal, c'est-à-dire selon la flèche F représentée sur cette figure. Simultanément à ce mouvement de translation, le chirurgien inculque à la clavette 8 un mouvement de rotation selon la flèche R représentée sur cette figure. Par ce mouvement, l'hélice 15 coopère avec les orifices 10 et assure un meilleur blocage au final de la tige 2 de liaison dans la pièce réceptacle 7.

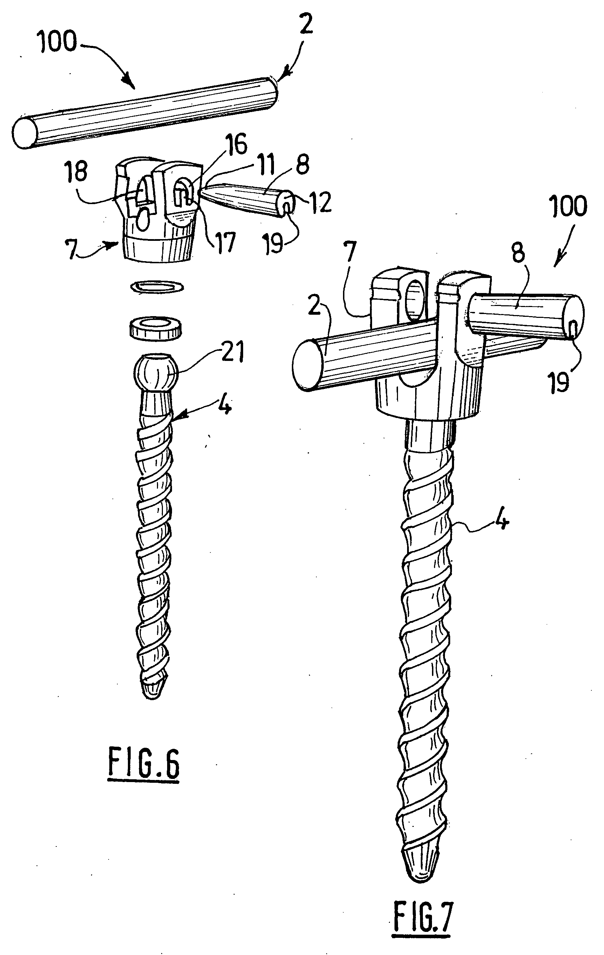

Sur les figures 6 à 8, est représenté une forme de réalisation du dispositif 1 selon l'invention, respectivement en perspective éclatée, en position ouverte et en position fermée. Dans l'exemple représenté sur ces figures, les moyens de blocage sont solidaires du dispositif. Une telle forme de réalisation permet au chirurgien de mettre en place le dispositif selon l'invention de façon plus rapide qu'avec les dispositifs de l'art antérieur. En effet, selon l'invention, le moyen de blocage fait partie du dispositif et le chirurgien ne manipule q'un seul outil.

Comme il apparaît sur la figure 6, la pièce réceptacle 7 comprend, sur la paroi d'une de ses branches du U, un premier orifice 16 en forme de demi disque, la base du demi disque étant pourvue d'un ergot 17. Elle comprend, sur la paroi de la deuxième branche du U, un deuxième orifice 18 en forme de demi disque, ce deuxième orifice 18 ne comportant pas d'ergot. Comme il apparaît plus nettement de la figure 5 et de la figure 6, la clavette 8 comprend, en deçà de son extrémité fine 11, une gorge longitudinale 19 s'étendant jusqu'à son extrémité large 12. Le départ de la gorge longitudinale 19 en deçà de l'extrémité fine 11 de la clavette 8 définit un arrêtoir 20 qui vient buter sur l'ergot 17 lorsque la clavette 8 est en position ouverte, comme montré sur la figure 7.

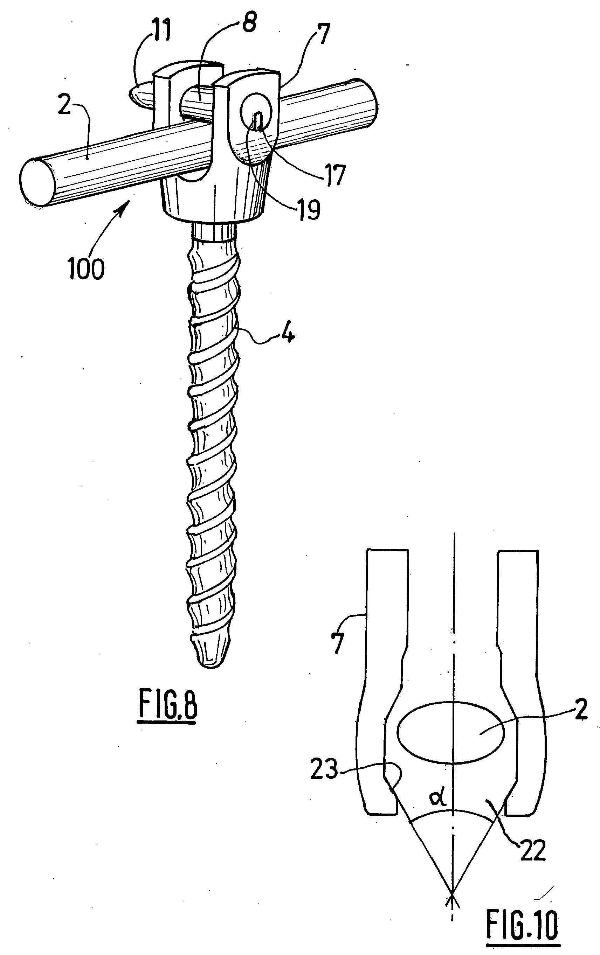

Ainsi, la gorge longitudinale 19 de la clavette 8 coopère avec l'ergot 17 dans l'orifice 16 de la paroi de la première branche du U afin de permettre le déplacement de la clavette 8 vers l'orifice 18 de la deuxième branche du U, et ce afin de pouvoir effectuer le blocage de la tige 2 de liaison, comme montré sur la figure 8, tout en empêchant le retour complet de ladite clavette 8 vers l'orifice de la paroi de la première branche du U et ainsi sa désolidarisation dudit dispositif 1, en particulier en position ouverte, comme montré sur la figure 7.

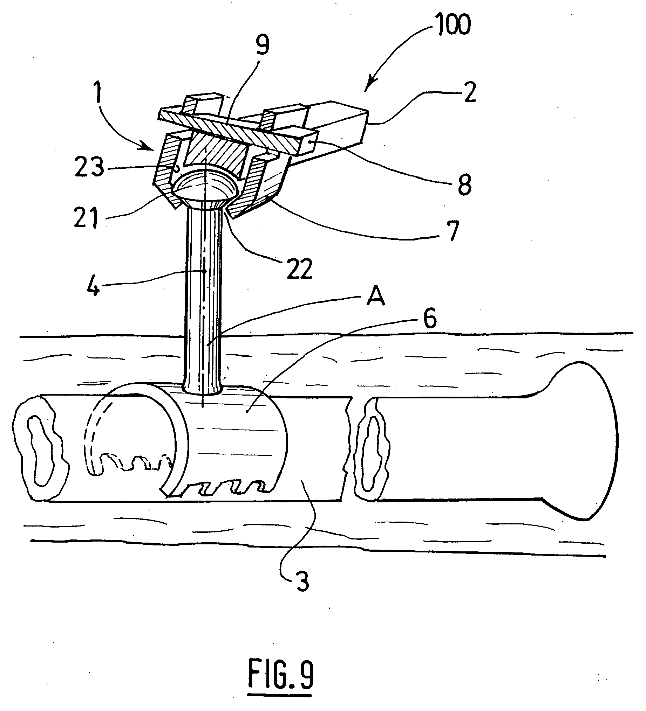

Le dispositif 1 des figures 6 à 8 et le dispositif 1 de la figure 9 comportent en outre une pièce réceptacle 7 qui est articulée sur le moyen d'ancrage, à savoir la vis 4. Comme il apparaît de la figure 6, la vis 4 comporte une tête sphérique 21. Le fond 23 de la pièce réceptacle 7 est conique et comprend un trou axial 22 visible sur les figures 9 et 10. Une vue en coupe de la pièce réceptacle 7 des dispositifs des figures 6 à 9 est représentée figure 10.

La tête sphérique 21 présente un diamètre strictement supérieur au diamètre du trou axial 22. Sur l'exemple représenté à la figure 10, le fond 23 de la pièce réceptacle 7 forme un cône d'angle α compris entre 15° et 60°, de préférence d'environ 50°. Cette tête sphérique 21 est engagée dans le fond 23 conique de la pièce réceptacle comme montré sur la figure 9.

Ainsi, dans une configuration mobile, comme représenté à la figure 9, la pièce réceptacle 7 est articulée sur la vis d'ancrage 4, de manière à permettre une orientation angulaire contrôlable dans les trois directions entre la vis 4 d'ancrage et la tige 2 de liaison.

Le chirurgien peut ainsi choisir très précisément l'orientation de la tige 2 de liaison avant de serrer la clavette 8 afin de procéder au blocage de ladite tige 2 de liaison. En particulier, grâce au coincement progressif et doux rendu possible par la surface inclinée 9 du moyen de blocage du dispositif 1 selon l'invention, le blocage est réversible et le chirurgien peut aisément desserrer la clavette 8, par exemple pour ajuster l'orientation de la tige 2 de liaison, puis ensuite resserrer cette clavette 8, et ce sans à-coups sur la vis d'ancrage 4 et donc au niveau de l'ancrage du dispositif 1 sur l'os ou fragment d'os 3.

De plus, de préférence, les branches du U de la pièce réceptacle 7 sont aptes à se défléchir entre une position non fléchie au repos et une position défléchie sous contrainte. Ainsi, ces branches autorisent, sous contrainte, le passage de la tige 2 de liaison vers le fond 23 de la pièce réceptacle 7, mais elles empêchent, au repos, la sortie de ladite tige 2 de liaison du fond 23 de la pièce réceptacle 7, comme montré sur la figure 10.

Sur la figure 9, le moyen d'ancrage 4 comporte un moyen de fixation 6 sur le fragment d'os 3, sous la forme d'un demi collier enserrant le fragment d'os 3.

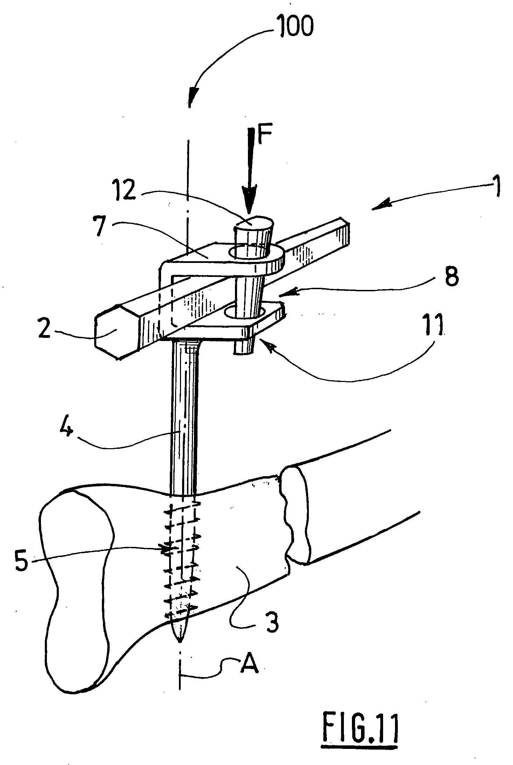

Sur la figure 11 est représentée un exemple d'un dispositif 1, dans lequel l'axe longitudinal du U de la pièce réceptacle 7 est perpendiculaire à l'axe longitudinal A de la vis d'ancrage 4. Dans ce cas, l'axe longitudinal de la clavette 8 est parallèle à l'axe longitudinal A de la vis d'ancrage 4 et la tige 2 de liaison est introduite dans la pièce réceptacle 7 perpendiculairement à l'axe longitudinal A de la vis 4 d'ancrage. Ce dispositif 1 fonctionne de la même façon que celui de la figure 1.

Sur la figure 12 est représentée la fixation de trois fragments d'os 3 à l'aide d'un ensemble 100 selon l'invention comprenant trois dispositifs 1 selon la présente invention et une tige 2 de liaison. Grâce aux dispositifs 1, la tige 2 de liaison est maintenue fermement bloquée, selon l'orientation voulue par le chirurgien. Il est ainsi par exemple possible de réduire les fractures de l'os représenté en ressoudant ses différents fragments.

Le dispositif selon l'invention est particulièrement avantageux dans le cas où l'opération de réduction de fracture d'un os nécessite, comme sur l'exemple de la figure 12, la mise en oeuvre de trois dispositifs. La facilité et la rapidité de la mise en place des dispositifs selon l'invention permet de réduire le temps d'intervention chirurgicale et donc de diminuer les risques associés, et de minimiser le traumatisme du patient.

Sur les figures 13 et 14 sont représentées des tiges 2 de liaison convenant aux dispositifs 1 et aux ensembles 100 selon l'invention.

Sur la figure 13 est représentée une tige 2 de liaison dont la section transversale 24 est sensiblement en forme de croissant, ladite tige 2 étant galbée. De préférence, une telle tige 2 est galbée en per-opératoire afin d'adapter sa forme à la morphologie du patient.

Sur la figure 14 est représentée une tige 2 de liaison rectiligne et présentant une section transversale 24 en forme de polygone.

Le dispositif 1 selon l'invention peut être utilisé pour souder les uns aux autres deux ou plusieurs petits os courts, tels que les os des poignets, des chevilles ou de la colonne vertébrale, comme les vertèbres, ou encore immobiliser plusieurs fragments d'un os long ou plusieurs os entre eux. Sa mise en place est particulièrement simple, rapide et fiable. En particulier, le dispositif 1 selon l'invention permet un blocage progressif et réversible de la tige 2 de liaison, sans à-coups et sans risque de déstabilisation du moyen d'ancrage du dispositif 1 au niveau de l'os ou des fragments d'os à relier entre eux.

Le dispositif et l'ensemble selon l'invention peuvent être aussi bien utilisés comme fixateurs internes, en tant qu'implants à l'intérieur du corps du patient, que comme fixateurs externes, la tête de la vis d'ancrage étant fixée dans l'os ou le fragment d'os et la pièce réceptacle étant à l'extérieur du corps du patient.

## Revendications

1. Dispositif (1) de fixation osseuse apte à immobiliser une tige (2) de liaison destinée à relier au moins un fragment d'os (3) à un autre, ledit dispositif (1) comprenant :
- au moins un moyen d'ancrage (4) destiné à être fixé sur ou dans ledit fragment d'os (3),
- au moins une pièce réceptacle (7), solidaire dudit moyen d'ancrage (4), destinée à recevoir au moins une partie de ladite tige (2) de liaison,
- des moyens de blocage (8) agencés pour bloquer ladite partie de ladite tige (2) de liaison au sein de ladite pièce réceptacle (7), lesdits moyens de blocage comprenant au moins un élément solide (8) présentant un axe longitudinal, ledit élément solide comprenant au moins une surface inclinée (9) apte, sous l'action d'une poussée dirigée selon l'axe longitudinal dudit élément solide, à créer par friction, un coincement progressif de ladite partie de tige (2) de liaison dans ladite pièce réceptacle (7), jusqu'au blocage complet de ladite partie de tige (2) de liaison dans ladite pièce réceptacle (7),
- ladite pièce réceptacle (7) étant en forme de U, ladite partie de tige (2) de liaison étant reçue au fond du U, la paroi de chaque branche du U étant pourvue d'un orifice (10),
- ledit élément solide étant sous la forme d'une clavette (8) dont la section transversale croît d'une première extrémité, appelée extrémité fine (11), à une deuxième extrémité, appelée extrémité large (12), le diamètre du cercle dans lequel est inscrit la section transversale de ladite extrémité large (12) étant strictement supérieur au diamètre du cercle inscrit dans ledit orifice (10),
- ladite clavette (8) étant apte à être introduite par son extrémité fine (11), successivement dans les orifices (10; 16, 18) des parois des branches du U de ladite pièce réceptacle (7), perpendiculairement à ladite tige (2) de liaison, et à être poussée selon son axe longitudinal jusqu'à entrer en contact avec ladite tige (2) de liaison et créer une friction entre ladite tige (2) de liaison et le fond de la pièce réceptacle (7),
- de telle sorte que, en position de blocage, ladite clavette (8) traverse chaque orifice (10) de chaque branche du U de ladite pièce réceptacle (7) et maintient ladite partie de tige (2) de liaison bloquée contre le fond de ladite pièce réceptacle (7), lesdits moyens de blocage (8) étant solidaires dudit dispositif (1), **caractérisé en ce que** ladite clavette (8) présente en deçà de son extrémité fine (11), une gorge longitudinale (19) s'étendant jusqu'à son extrémité large (12), apte à coopérer avec un ergot (17) ménagé dans l'orifice (16) de la paroi d'une première branche du U, afin de permettre le déplacement de ladite clavette (8) vers l'orifice (18) de la paroi de la deuxième branche du U et d'empêcher le retour complet de ladite clavette (8) vers l'orifice (16) de la paroi de la première branche du U et ainsi la désolidarisation de cette dernière (8) dudit dispositif (1).

2. Dispositif (1) selon la revendication précédente, **caractérisé en ce que** le périmètre de la section transversale de ladite clavette (8) présente une forme choisie parmi un cercle, un polygone, ou une combinaison d'arcs de cercle et/ou de segments.

3. Dispositif (1) selon la revendication 1 ou 2, **caractérisé en ce que** ladite clavette (8) présente, sur sa partie destinée à être en contact avec ladite tige de liaison en position de blocage, des structures en creux ou en reliefs, telles que des aspérités, des rainures (13, 14) et/ou des hélices (15), aptes à sécuriser le blocage de ladite tige de liaison au sein de ladite pièce réceptacle.

4. Dispositif (1) selon l'une quelconque des revendications précédentes, **caractérisé en ce que**, dans une configuration dite mobile, ladite pièce réceptacle (7) est articulée sur ledit moyen d'ancrage (4), de manière à permettre une orientation angulaire contrôlable dans les trois directions entre ledit moyen d'ancrage (4) et ladite tige (2) de liaison.

5. Dispositif (1) selon la revendication précédente, **caractérisé en ce que** ladite pièce réceptacle (7) présente un fond (23) conique percé d'un trou axial (22),
- ledit moyen d'ancrage est sous la forme d'une vis (4) comprenant une tête sphérique (21) de diamètre strictement supérieur au diamètre dudit trou axial (22), ladite tête sphérique (21) étant engagée dans le fond (23) conique.

6. Dispositif (1) selon la revendication précédente, **caractérisé en ce que** ledit fond (23) conique forme un cône d'angle α compris entre 15° et 60°.

7. Dispositif (1) selon l'une quelconque des revendications précédentes, **caractérisé en ce que** lesdites branches du U sont aptes à se défléchir entre une position non fléchie au repos et une position défléchie sous contrainte, afin d'autoriser, sous contrainte, le passage de la tige (2) de liaison vers le fond (23) de la pièce réceptacle (7), et d'empêcher, au repos, la sortie de ladite tige (2) de liaison du fond (23) de la pièce réceptacle (7).

8. Dispositif selon l'une quelconque des revendications précédentes, **caractérisé en ce que** ledit moyen d'ancrage étant sous la forme d'une vis (4) allongée, l'axe longitudinal du U de ladite pièce réceptacle (7) est sensiblement parallèle à, et de préférence se confond avec, l'axe longitudinal A de ladite vis (4) allongée.

9. Dispositif selon l'une quelconque des revendications 1 à 7, **caractérisé en ce que** ledit moyen d'ancrage étant sous la forme d'une vis (4) allongée, l'axe longitudinal du U de ladite pièce réceptacle (7) est sensiblement perpendiculaire avec l'axe longitudinal A de ladite vis (4) allongée.

10. Ensemble (100) pour fixation osseuse **caractérisé en ce qu'**il comprend au moins un dispositif (1) selon l'une quelconque des revendications 1 à 12 et une tige (2) de liaison.

11. Ensemble (100) selon la revendication précédente, **caractérisé en ce que** ladite tige (2) de liaison présente une section transversale (24) sensiblement en forme de croissant, ladite tige (2) étant galbée.

12. Ensemble (100) selon la revendication 10, **caractérisé en ce que** ladite tige (2) de liaison est rectiligne et présente une section transversale (24) en forme de polygone.

## Patentansprüche

1. Vorrichtung (1) zur Knochenfixation, die geeignet ist, einen Verbindungsschaft (2) stillzustellen, der dazu bestimmt ist, mindestens ein Knochenfragment (3) mit einem anderen zu verbinden, wobei die Vorrichtung (1) Folgendes umfasst:
- mindestens ein Verankerungsmittel (4), das dazu bestimmt ist, auf oder in dem Knochenfragment (3) befestigt zu werden,
- mindestens ein Aufnahmeteil (7), das fest mit dem Verankerungsmittel (4) verbunden ist, das dazu bestimmt ist, mindestens einen Teil des Verbindungsschafts (2) aufzunehmen,
- Blockierungsmittel (8), die eingerichtet sind, um den Teil des Verbindungsschafts (2) im Inneren des Aufnahmeteils (7) zu blockieren,
wobei die Blockierungsmittel mindestens ein festes Element (8) umfassen, das eine Längsachse aufweist, wobei das feste Element mindestens eine schräge Oberfläche (9) umfasst, die geeignet ist, unter der Einwirkung eines Schubs, der entlang der Längsachse des festen Elements ausgerichtet ist, durch Reibung ein allmähliches Verklemmen des Teils des Verbindungsschafts (2) in dem Aufnahmeteil (7) bis zum kompletten Blockieren des Teils des Verbindungsschafts (2) in dem Aufnahmeteils (7) zu schaffen,
- wobei das Aufnahmeteil (7) U-Form hat, wobei der Teil des Verbindungsschafts (2) im Grund des U aufgenommen ist, wobei die Wand jedes Schenkels des U mit einer Öffnung (10) versehen ist,
- wobei das feste Element die Form eines Keils (8) hat, dessen Querschnitt von einem ersten Ende, feines Ende (11) genannt, zu einem zweiten Ende, breites Ende (12) genannt, zunimmt, wobei der Durchmesser des Kreises, in den der Querschnitt des breiten Endes (12) fällt, strikt größer ist als der Durchmesser des Kreises ist, der in der Öffnung (10) liegt,
- wobei der Keil (8) geeignet ist, durch sein feines Ende (11) nacheinander in die Öffnungen (10; 16, 18) der Wände der Schenkel des U des Aufnahmeteils (7) senkrecht zu dem Verbindungsschaft (2) eingefügt und entlang seiner Längsachse geschoben zu werden, bis er mit dem Verbindungsschaft (2) in Berührung tritt und eine Reibung zwischen dem Verbindungsschaft (2) und dem Grund des Aufnahmeteils (7) schafft,
- derart, dass der Keil (8) in Blockierungsposition jede Öffnung (10) jedes Schenkels des U des Aufnahmeteils (7) durchquert und den Teil des Verbindungsschafts (2) gegen den Grund des Aufnahmeteils (7) blockiert hält, wobei die Blockierungsmittel (8) fest mit der Vorrichtung (1) verbunden sind, **dadurch gekennzeichnet, dass** der Keil (8) über sein feines Ende (11) hinaus eine Längshohlkehle (19) aufweist, die sich bis zu seinem breiten Ende (12) erstreckt, die geeignet ist, mit einem Dorn (17), der in der Öffnung (16) der Wand eines ersten Schenkels des U angelegt ist, zusammenzuwirken, um das Bewegen des Keils (8) zu der Öffnung (18) der Wand des zweiten Schenkels des U zu erlauben und das komplette Zurückkehren des Keils (8) zu der Öffnung (16) der Wand des ersten Schenkels des U und daher das Trennen dieser Letzteren (8) von der Vorrichtung (1) zu verhindern.

2. Vorrichtung (1) nach dem vorhergehenden Anspruch, **dadurch gekennzeichnet, dass** der Umkreis des Querschnitts des Keils (8) eine Form aufweist, die aus einem Kreis, einem Vieleck oder einer Kombination von Kreisbögen und/oder Kreissegmenten ausgewählt ist.

3. Vorrichtung (1) nach Anspruch 1 oder 2, **dadurch gekennzeichnet, dass** der Keil (8) auf seinem Teil, der dazu bestimmt ist, mit dem Verbindungsschaft in Blockierungsposition in Berührung zu sein, Strukturen in Vertiefungen oder Reliefs, wie zum Beispiel Rauheiten, Rillen (13, 14) oder Schrauben (15) aufweist, die geeignet sind, um das Blockieren des Verbindungsschafts innerhalb des Aufnahmeteils zu sichern.

4. Vorrichtung (1) nach einem der vorhergehenden Ansprüche, **dadurch gekennzeichnet, dass** in einer sogenannten beweglichen Konfiguration das Aufnahmeteil (7) auf dem Verankerungsmittel (4) derart angelenkt ist, dass es eine winkelige Ausrichtung, die in die drei Richtungen zwischen dem Verankerungsmittel (4) und dem Verbindungsschaft (2) steuerbar ist, erlaubt.

5. Vorrichtung (1) nach dem vorhergehenden Anspruch, **dadurch gekennzeichnet, dass** das Aufnahmeteil (7) einen kegeligen Grund (23), der mit einer axialen Bohrung (22) durchbohrt ist, aufweist,
- wobei das Verankerungsmittel die Form einer Schraube (4) hat, die einen Kugelkopf (21) mit Durchmesser strikt größer als der Durchmesser der axialen Bohrung (22) umfasst, wobei der Kugelkopf (21) in den kegeligen Grund (23) eingefügt ist.

6. Vorrichtung (1) nach dem vorhergehenden Anspruch, **dadurch gekennzeichnet, dass** der kegelige Grund (23) einen Kegel mit einem Winkel α zwischen 15° und 60° bildet.

7. Vorrichtung (1) nach einem der vorhergehenden Ansprüche, **dadurch gekennzeichnet, dass** die Schenkel des U geeignet sind, sich zwischen einer nicht gebogenen Position in Ruhestellung und einer unter Belastung gebogenen Position gerade zu richten, um unter Belastung das Durchgehen des Verbindungsschafts (2) zu dem Grund (23) des Aufnahmeteils (7) zu gestatten und in Ruhestellung das Austreten des Verbindungsschafts (2) aus dem Grund (23) des Aufnahmeteils (7) zu verhindern.

8. Vorrichtung nach einem der vorhergehenden Ansprüche, **dadurch gekennzeichnet, dass**, da das Verankerungsmittel die Form einer länglichen Schraube (4) hat, die Längsachse des U des Aufnahmeteils (7) im Wesentlichen mit der Längsachse A der länglichen Schraube (4) parallel ist und vorzugsweise mit ihr zusammenfällt.

9. Vorrichtung nach einem der Ansprüche 1 bis 7, **dadurch gekennzeichnet, dass** das Verankerungsmittel die Form einer länglichen Schraube (4) hat, wobei die Längsachse des U des Aufnahmeteils (7) im Wesentlichen zu der Längsachse A der länglichen Schraube (4) senkrecht ist.

10. Baugruppe (100) zur Knochenfixation, **dadurch gekennzeichnet, dass** sie mindestens eine Vorrichtung (1) nach einem der Ansprüche 1 bis 12 und einen Verbindungsschaft (2) umfasst.

11. Baugruppe (100) nach dem vorhergehenden Anspruch, **dadurch gekennzeichnet, dass** der Verbindungsschaft (2) einen Querschnitt (24) im Wesentlichen in Croissant-Form aufweist, wobei der Schaft (2) gewölbt ist.

12. Baugruppe (100) nach Anspruch 10, **dadurch gekennzeichnet, dass** der Verbindungsschaft (2) geradlinig ist und einen vieleckigen Querschnitt (24) aufweist.

## Claims

1. A bone fixation device (1) capable of immobilizing a connecting rod (2) intended to connect at least one bone fragment (3) to another one, said device (1) comprising :
- at least one anchoring means (4) intended to be fixed on or inside said bone fragment (3),
- at least one container part (7), secured to said anchoring means (4), intended to receive at least one portion of said connecting rod (2),
- blocking means (8) arranged to block said portion of said connecting rod (2) within said container part (7),
said blocking means comprising at least one solid element (8) presenting a longitudinal axis, said solid element comprising at least one tilted surface (9) capable of creating through friction, when pushed along the longitudinal axis of said solid element, a progressive sticking of said portion of the connecting rod (2) inside said container part (7), until complete blockage of said connecting rod (2) portion inside said container part (7),
- said container part (7) being U-shaped, said connecting rod (2) portion being received at the bottom of the U-shape, the wall of each leg of the U-shape being provided with an orifice (10),
- said solid element being in the form of a key (8) the cross-section of which increases starting from a first end, called thin end (11), toward a second end, called large end (12), the diameter of the circle in which the cross-section of said large end (12) is inscribed being strictly larger than the diameter of the circle inscribed within said orifice (10),
- said key (8) being capable of being inserted, by its thin end (11), successively inside the orifices (10 ; 16, 18) of the walls of the legs of the U-shape of the container part (7), perpendicular to said connecting rod (2), and to be pushed along its longitudinal axis until coming into contact with said connecting rod (2) and creating a friction between said connecting rod (2) and the bottom of the container part (7),
- so that, in the blocking position, said key (8) passes through each orifice (10) of each leg of the U-shape of said container part (7) and holds said connecting rod (2) portion blocked against the bottom of said container part (7), said blocking means (8) being secured to said device (1), **characterized in that** said key (8) presents, below its thin end (11), a longitudinal channel (19) extending to its large end (12), capable of cooperating with a lug (17) arranged in the orifice (16) of the wall of a first leg of the U-shape, in order to enable the displacement of said key (8) toward the orifice (18) of the wall of the second leg of the U-shape and to prevent the complete return of said key (8) toward the orifice (16) of the wall of the first leg of the U-shape and thereby the separation of the latter (8) from said device (1).

2. The device (1) according to the preceding claim, **characterized in that** the perimeter of the cross-section of said key (8) presents a shape selected from a circle, a polygon, or a combination of circular arcs and/or segments.

3. The device (1) according to claim 1 or 2, **characterized in that** said key (8) presents, on its portion intended to be in contact with said connecting rod at the blocking position, recessed or protruding structures, such as asperities, grooves (13, 14) and/or helixes (15), capable of securing the blockage of said connecting rod within said container part.

4. The device (1) according to any one of the preceding claims, **characterized in that**, in a configuration called movable configuration, said container part (7) is hinged on said anchoring means (4), so as to allow a controllable angular orientation in the three directions between said anchoring means (4) and said connecting rod (2).

5. The device (1) according to the preceding claim, **characterized in that** said container part (7) presents a conical bottom (23) pierced with an axial hole (22),
- said anchoring means is in the form of a screw (4) comprising a ball head (21) with a diameter strictly larger than the diameter of said axial hole (22), said ball head (21) being engaged in the conical bottom (23).

6. The device (1) according to the preceding claim, **characterized in that** said conical bottom (23) forms a cone with an angle α comprised between 15° and 60°.

7. The device (1) according to any one of the preceding claims, **characterized in that** said legs of the U-shape are capable of deflecting between an unflexed position at rest and a deflected position under stress, in order to allow, under stress, the connecting rod (2) to pass to the bottom (23) of the container part (7), and to prevent, at rest, said connecting rod (2) from coming out from the bottom (23) of the container part (7).

8. The device according to any one of the preceding claims, **characterized in that**, as said anchoring means is in the form of an elongated screw (4), the longitudinal axis of the U-shape of said container part (7) is substantially parallel to, and preferably coincident with, the longitudinal axis A of said elongated screw (4).

9. The device according to any one of claims 1 to 7, **characterized in that**, as said anchoring means is in the form of an elongated screw (4), the longitudinal axis of the U-shape of said container part (7) is substantially perpendicular to the longitudinal axis A of said elongated screw (4).

10. A bone fixation set (100) **characterized in that** it comprises at least one device (1) according to anyone of claims 1 to 12 and a connecting rod (2).

11. The set (100) according to the preceding claim, **characterized in that** said connecting rod (2) presents a substantially crescent-shaped cross-section (24), said rod (2) being curved.

12. The set (100) according to claim 10, **characterized in that** said connecting rod (2) is rectilinear and presents a polygon-shaped cross-section (24).
